# EUROPÄISCHE PATENTANMELDUNG

(11) **EP 3 730 165 A1**
(43) Veröffentlichungstag der Anmeldung: **28.10.2020**
(21) Anmeldenummer: 19171192.8
(22) Anmeldetag: 25.04.2019
(51) Int. Cl.: A61M 1/00, H01R 13/22, H01R 13/62

(54) **MOTORGETRIEBENE MEDIZINISCHE SAUGPUMPE UND VERFAHREN ZUM ANSCHLIESSEN EINER SOLCHEN SAUGPUMPE AN EINE STROMQUELLE**

(71) Anmelder: Medela Holding AG, 6340 Baar (CH)
(72) Erfinder: HONEGGER, Adrian, 6004 Luzern (CH); GIEZENDANNER, Charles, 6443 Morschach (CH)
(74) Vertreter: Grünecker Patent- und Rechtsanwälte PartG mbB

(57) **Zusammenfassung**

Die vorliegende Erfindung betrifft eine motorgetriebene Saugpumpe und ein Verfahren zum Anschließen einer solchen. Die Saugpumpe hat ein Pumpaggregat zur Erzeugung eines Unterdrucks, das in einem Gehäuse (2) aufgenommen ist. Es ist ferner ein Anschlussgehäuse (10) vorgesehen, das über ein Anschlusskabel (30) an eine Stromquelle anschließbar ist und Kontaktelemente (16, 18) zur Bestromung des Pumpaggregats mit einem Leistungsstrom aufweist. Die Kontaktelemente (16, 18) sind bei an dem Gehäuse (2) gekoppelten Anschlussgehäuse (10) elektrisch leitend mit gehäuseseitig vorgesehenen und zu dem Pumpaggregat führenden Kontaktgegenelementen (46, 48) verbunden. Zur Erhöhung der elektrischen Sicherheit ist ein Sensor (22), eine Auswerteinheit (32) und ein Schalter (34) vorgesehen. Der Sensor (22) ist zur Überprüfung der Kopplung des Anschlussgehäuses (10) eingerichtet. Der Schalter (34) schaltet eine Leistungsstromzufuhr zu zumindest einem der Kontaktelemente (16, 18). Die Auswerteinheit (32) ist datenmäßig mit dem Sensor (22) und dem Schalter (34) verbunden, und zwar derart, dass der Schalter bei einem die Kopplung bestätigenden Signal des Sensors (22) das Kontaktelement (16, 18) mit der Leistungsstromzufuhr verbindet und beim Ausbleiben eines die Kopplung bestätigenden Signals des Sensors (22) die Leistungsstromzufuhr trennt. Bei dem erfindungsgemäßen Verfahren wird zunächst zumindest ein stromlos geschaltetes Kontaktelement (16, 18) elektrisch mit dem zur Bestromung des Pumpaggregates vorgesehenen gehäuseseitigen Kontaktgegenelement (46, 48) elektrisch verbunden. Danach wird aufgrund eines von dem die entsprechende Kontaktierung bestätigenden Signals der Leistungsstrom auf das Kontaktelement (16, 18) aufgeschaltet.

## Beschreibung

Die vorliegende Erfindung betrifft eine motorgetriebene medizinische Saugpumpe mit einem Pumpaggregat zur Erzeugung eines Unterdrucks, das in einem Gehäuse aufgenommen ist, und mit einem mit dem Gehäuse mechanisch koppelbaren Anschlussgehäuse, das über ein Anschlusskabel an eine Stromquelle anschließbar ist. Das Anschlussgehäuse hat dazu Kontaktelemente zur Bestromung des Pumpaggregats mit einem Leistungsstrom. Bei an dem Gehäuse gekoppelten Anschlussgehäuse sind diese Kontaktelemente elektrisch leitend mit gehäuseseitig vorgesehenen Kontaktgegenelementen verbunden, die zu dem Pumpaggregat führen.

Eine solche motorgetriebene Pumpe ist beispielsweise als Exsudatpumpe bekannt. Eine andere Ausgestaltung einer entsprechenden Saugpumpe ist eine Brustpumpe zum Absaugen von Muttermilch der weiblichen Brust. Dabei erfolgt der elektrische Anschluss an eine Stromquelle durch ein steckkontaktierbares Kabel, welches ein Anschlussgehäuse aufweist. In diesem Anschlussgehäuse sind üblicherweise die Kontaktelemente so verbaut, dass diese nicht an der Außenseite frei liegen. Die Kontaktelemente sind üblicherweise als weibliche Steckkontaktelemente vorgesehen, wobei das Gehäuse zugeordnete männliche Steckkontaktgegenelemente aufweist, die zur Steckkontaktierung zunächst ein Stück weit in eine Höhlung des Anschlussgehäuses eingeführt werden müssen, bis ein elektrischer Kontakt zwischen den Kontaktelementen und den Kontaktgegenelementen erzeugt wird.

Die zuvor beschriebene allgemein bekannte Anordnung der Kontaktelemente in dem Anschlussgehäuse dient insbesondere der elektrischen Sicherheit. So wird verhindert, dass bei der üblichen Handhabung des Anschlussgehäuses die dort anliegende Spannung unbeabsichtigt übertragen werden kann. Allerdings sieht die vorliegende Erfindung die Notwendigkeit, die elektrische Sicherheit insofern zu verbessern.

So wird mit der vorliegenden Erfindung eine motorgetriebene medizinische Saugpumpe mit den Merkmalen von Anspruch 1 vorgeschlagen.

Bei der erfindungsgemäßen motorgetriebenen medizinischen Saugpumpe sind ein Sensor, eine Auswerteinheit und ein Schalter vorgesehen. Der Sensor ist zur Überprüfung der Kopplung des Anschlussgehäuses eingerichtet. Der Sensor kann hierfür ein berührungsloser oder ein berührender Sensor sein. Der Sensor ist so ausgebildet, dass er die Kopplung, d.h. die richtige Kopplung des Anschlussgehäuses an dem Gehäuse, erkennt. Als richtige Kopplung ist dabei diejenige Kopplung des Anschlussgehäuses an dem Gehäuse zu verstehen, bei welcher die Kontaktelemente mit den zugeordneten Kontaktgegenelementen elektrisch verbunden sind. So reicht für die Erkennung der richtigen Kopplung nicht eine irgendwie geartete Anbindung des Anschlussgehäuses an dem Gehäuse. Vielmehr muss das Anschlussgehäuse exakt so gegenüber dem Gehäuse positioniert sein, dass der elektrische Kontakt zwischen dem Anschlussgehäuse und dem Gehäuse verwirklicht ist. Die Auswerteinheit ist mit dem Sensor und dem Schalter datenmäßig verbunden. Der Schalter schaltet den Leistungsstrom. Dafür befindet sich der Schalter üblicherweise in dem Anschlussgehäuse oder einem zur Steckkontaktierung mit dem Versorgungsstrom auf der anderen Seite eines Anschlusskabels verbundenen Anschlussstecker. Durch die datenmäßige Verbindung der Auswerteinheit mit dem Sensor und dem Schalter ist es danach möglich, das von dem Sensor abgegebene Sensorsignal zu analysieren und durch den Schalter den Leistungsstrom auf das, beziehungsweise die Kontaktelemente aufzuschalten, die an dem Anschlussgehäuse befestigt sind.

Mit der vorliegenden Erfindung wird dementsprechend eine Lösung vorgeschlagen, bei welcher das, beziehungsweise sämtliche stromführende Kontaktelemente des Anschlussgehäuses so lange von dem Leistungsstrom getrennt sind, bis eine mechanische Kopplung zwischen dem Anschlussgehäuse und dem Gehäuse detektiert wird. So lässt sich das Anschlussgehäuse mit den daran vorgesehenen Kontaktelementen handhaben, ohne dass die Gefahr eines Stromschlags gegeben ist, selbst wenn der Anschlussstecker beziehungsweise das zu dem Anschlussgehäuse führende Anschlusskabel, welches dauerhaft mit der Versorgungsspannung verbunden sein kann, unter Spannung steht.

Die vorliegende Erfindung lässt sich in vielen Bereichen nutzen, in denen es auf eine gute elektrische Sicherheit ankommt, beziehungsweise nicht ausgeschlossen werden kann, dass Elemente, Extremitäten oder Medien in Kontakt mit den Kontaktelementen gelangen. So eignet sich die vorliegende Erfindung insbesondere auch zum Anschluss eines elektrischen Verbrauchers in einem Feuchtraum oder in einem Bereich, in dem Dämpfe oder Gase, insbesondere elektrisch leitende Gase oder explosive Gase vorhanden sind. Die erfindungsgemäße Lösung eignet sich grundsätzlich für jede elektrische Kontaktierung zu einem elektrischen Verbraucher, der in einem Gehäuse vorgesehen ist. Das Anschlussgehäuse ist mit diesem Gehäuse koppelbar und weist die Kontaktelemente zur Bestromung des Verbrauchers mit dem Leistungsstrom auf, um bei an dem Gehäuse gekoppeltem Anschlussgehäuse elektrisch leitend mit dem gehäuseseitig vorgesehenen und zu dem Verbraucher führenden Kontaktelementen verbunden zu werden. Die elektrische Kontaktierung hat einen Sensor, eine Auswerteinheit und einen Schalter wie zuvor beschrieben. Soweit in der Beschreibung der Erfindung darauf abgestellt wird, dass der Leistungsstrom dem Pumpaggregat in dem Gehäuse zugeleitet wird, bedeutet dies nicht, dass der Leistungsstrom nicht auch an andere Komponenten innerhalb des Gehäuses weitergeleitet wird. Das Gehäuse der erfindungsgemäßen Saugpumpe nimmt vielmehr üblicherweise auch eine eine Steuervorrichtung verwirklichende Elektronik, die den Leistungsstrom steuert bzw. schaltet, sowie üblicherweise einen Akkumulator in sich auf, über den die in das Gehäuse eingeleitete elektrische Energie gespeichert werden kann.

Wie oben dargelegt, kann der Sensor jeder beliebige Sensor sein, der die mechanische Kopplung, insbesondere die richtige mechanische Kopplung des Anschlussgehäuses an dem Gehäuse detektiert. Der Sensor kann ein magnetischer Schalter, ein Näherungssensor oder ein optischer Sensor sein. Der Sensor sollte zumindest an dem Anschlussgehäuse vorgesehen sein, um die Annährung und mechanische Kopplung des Anschlussgehäuses an dem Gehäuse zu detektieren und um unmittelbar mit der anschlussseitig vorgesehenen Auswerteinheit zu kommunizieren.

Eine robuste und zuverlässige Lösung wird gemäß einer bevorzugten Weiterbildung der vorliegenden Erfindung vorgeschlagen, nach welcher der Sensor ein Sensorelement aufweist, dem gehäuseseitig ein Sensorgegenelement zugeordnet ist, wobei das Sensorelement abhängig von der relativen Lage zu dem Sensorgegenelement ein die Kopplung bestätigendes Signal abgibt. Zumindest eines der Kontaktelemente und das Sensorelement sind so in dem Anschlussgehäuse angeordnet, dass bei mechanischer Kopplung des Anschlussgehäuses an dem Gehäuse zunächst das Kontaktelement mit dem zugeordneten Kontaktgegenelement elektrisch verbunden und erst danach das Sensorelement relativ zu dem Sensorgegenelement so positioniert wird, dass ein die Kopplung bestätigendes Signal abgesetzt wird, so dass der Leistungsstrom aufgeschaltet wird. Bei dieser bevorzugten Weiterbildung sind danach Sensorelement und Sensorgegenelement einander so zugeordnet, dass bevorzugt deren elektrische Kontaktierung das Sensorsignal absetzt. Ein Sensorelement ist in der einfachsten Ausgestaltung ein elektrischer Pin, der mit einer durch das Sensorgegenelement ausgebildeten Kontaktfläche zusammenwirkt.Bei einer einfachen Ausgestaltung ist das Sensorelement und/oder das Sensorgegenelement als nachlaufendes Element ausgebildet, welches in Ankoppelrichtung des Anschlussgehäuses beim mechanischen Koppeln desselben an dem Gehäuse von dem Anschlussgehäuse bzw. gehäuseseitig abragt, indes in eben dieser Ankoppelrichtung hinter dem Kontaktelement bzw. Kontaktgegenelement angeordnet ist. Dabei wird das Kontaktelement und/oder das Kontaktgegenelement durch eine Feder in Ankoppelrichtung vorgespannt und beweglich gehalten sein, um nach ihrer Kontaktierung den noch zurückzulegenden Weg des Anschlussgehäuses in Richtung auf das Gehäuse zuzulassen, bis das Anschlussgehäuses an dem Gehäuse mechanisch gekoppelt und/oder das Sensorelement und das Sensorgegenelement elektrisch miteinander verbunden sind. Das Sensorelement und Sensorgegenelement können in entsprechender Weise federvorgespannt und beweglich sein, um trotz gegebener Toleranzen und Ungenauigkeiten bei der mechanischen Kopplung von Anschlussgehäuse und Gehäuse eine sichere Kontaktierung zu erlauben.

Als Feder in dem vorerwähnten Sinn kann jedes elastische Element angesehen werden, welches eine Rückstellkraft durch Formänderung aufnehmen und speichern kann. Bedeutsam ist lediglich, dass das Kontaktelement und/oder das Kontaktgegenelement beziehungsweise das Sensorelement und das Sensorgegenelement im gekoppelten Zustand mit einer gewissen Vorspankraft ineinandergreifen und/oder gegeneinander liegen.

Die Kontaktgegenelemente sind bevorzugt fest und unbeweglich in dem Gehäuse beziehungsweise relativ zu diesem positioniert. Die Ausgleichbewegung wird bevorzugt allein durch bewegliche Kontaktelemente auf Seiten des Anschlussgehäuses bewirkt.

Die vorliegende Erfindung lässt sich insbesondere von der Überlegung leiten, dass mit der Möglichkeit einer Zuschaltung des Leistungsstromes erst nach einer erkannten Positionierung des Anschlussgehäuses an dem Gehäuse neue Möglichkeiten der Kontaktierung gegeben sind. Denn mit der erfindungsmäßen Lösung ist es nicht notwendig, die üblicherweise unter Spannung stehenden Kontaktelemente mechanisch vor Berührung zu schützen. So ist es beispielsweise mit der vorliegenden Erfindung möglich, das Anschlussgehäuse und/oder das Gehäuse zumindest im Bereich der Kontaktierung mit einer absatzfreien Anlagefläche auszubilden, an der das jeweils andere von Anschlussgehäuse und Gehäuse im gekoppelten Zustand anliegt. Die Anlagefläche ist danach diejenige Fläche, über welche sich das Anschlussgehäuse und das Gehäuse in gekoppeltem Zustand berühren. Innerhalb dieser Anschlussfläche befinden sich üblicherweise die Kontaktelemente. Jedenfalls bietet die Weiterbildung die Möglichkeit, das Anschlussgehäuse und/oder das Gehäuse so auszubilden, dass sich auch im Bereich der Übertragung des Leistungsstromes in das Gehäuse keine Grate und andere Kante beziehungsweise Hinterschnitte zeigen, an denen sich Verschmutzung einnisten kann. Die Weiterbildung schafft somit die Möglichkeit, das Gehäuse beziehungsweise das Anschlussgehäuse so auszubilden, dass es sich leicht reinigen und/oder desinfizieren lässt. So eignet sich die Weiterbildung insbesondere für die Übertragung des Leistungsstromes an ein Gehäuse im medizinischen Bereich.

Die zuvor diskutierte Weiterbildung verzichtet auch auf mechanische Formschlusselemente, mit denen das Anschlussgehäuse und das Gehäuse mechanisch miteinander verbunden und gekoppelt werden. Zur Kompensation werden mit der vorliegenden Erfindung berührungsfreie Haltemittel vorgeschlagen, über welche das Anschlussgehäuse in gekoppeltem Zustand an dem Gehäuse gehalten sind. Bevorzugt ist dabei das Anschlussgehäuse an dem Gehäuse mittels Magnetkraft gehalten. Dazu können Permanentmagneten, ein magnetisierbares Material und/oder Elektromagneten zum Einsatz kommen. Geeignet sind sämtliche Lösungen, die beispielsweise aus dem Stand der Technik EP 2 287 972 B1, CN 207 853 072 U oder CN 202 050 089 U zur mechanischen Kopplung von Anschlussgehäuse und Gehäuse zur Übertragung eines Leistungsstromes bekannt sind.

Die erfindungsgemäße Lösung ist nicht auf eine bestimmte Voltage des Leistungsstromes beschränkt. Allerdings wird insbesondere an Ausgestaltungen gedacht, um Verbraucher an die üblichen Stromquellen in einem Gebäude, beispielsweise einem Krankenhaus, einem Altenheim oder einem Wohnhaus anzuschließen. So wird üblicherweise die elektrische Kontaktierung der vorliegenden Erfindung eine Spannung von nicht mehr als 230 Volt übertragen. Dabei kann der zwischen den Kontaktelementen und den Kontaktgegenelementen übertragene Leistungsspannung auch auf Niedervolt transformiert sein. Insbesondere medizinische Saugpumpen werden üblicherweise mit einem Aggregat ausgestattet, das mit 12 Volt betrieben werden kann, um die Möglichkeit zu schaffen, die Saugpumpe auch über eine autonome, üblicherweise in dem Gehäuse verbaute Energiequelle, eine Batterie oder einen wiederaufladbaren Akkumulator anzutreiben. Auch bei solchen Niederspannungslösungen erweist sich die erfindungsgemäße Ausgestaltung der elektrischen Kontaktierung als vorteilhaft, da insbesondere im medizinischen Bereich auch geringste Stromstöße an Patienten oder Säuglingen zu vermeiden sind. So eignet sich die erfindungsgemäße elektrische Kontaktierung insbesondere für jeglichen Anschluss von einem Verbraucher in einem medizinaltechnischen, elektrisch betriebenen Gerät an einen externen über ein Anschlusskabel zugeleiteten Leistungsstrom.

Bei der hier zu diskutierenden Weiterbildung der mechanischen Kopplung über eine Magnetkraft kann eine exakte Lagepositionierung des angekoppelten Anschlussgehäuses durch ein einziges Magnetelement erfolgen, das zusammen mit einem einzigen gehäuseseitigen Magnetgegenelement das Anschlussgehäuse in vorbestimmter Ausrichtung an dem Gehäuse hält. Das Magnetelement bzw. das Magnetgegenelement sollten dazu eine asymmetrische Gestalt und/oder asymmetrische Anordnung relativ zu einer Symmetrieachse einer Mulde haben, die das Anschlussgehäuse in sich aufnimmt und gegebenenfalls mit gewissem Spiel endseitig umgibt, wobei die Grundfläche des Anschlussgehäuses und die Grundfläche der Mulde einander zumindest in etwa entsprechen sollten. Auch die Mulde und/oder das Anschlussgehäuse selbst können eine spezifische Kontur haben, welche eine vorbestimmte Ausrichtung des Anschlussgehäuses in der Mulde vorgibt.

Bevorzugt sind an dem Anschlussgehäuse zumindest zwei Magnetelemente vorgesehen, die mit unterschiedlichen Polen versehen sind und im gekoppelten Zustand zur eindeutigen Positionierung des Anschlussgehäuses relativ zu dem Gehäuse wirksam sind. Die zumindest zwei Magnetelemente stellen danach sicher, dass das Anschlussgehäuse in mechanisch gekoppeltem Zustand in der richtigen, eineindeutig durch die zumindest zwei Magnetelemente vorgegebenen Weise an der gehäuseseitigen Anlagefläche anliegt. Werden die Magnetelemente mit unterschiedlichen Polen in falscher Ausrichtung an die üblicherweise gehäuseseitig in einer Mulde vorgesehene Anlagefläche angelegt, ergeben sich aufgrund von gehäuseseitigen Gegenmagneten abstoßende Magnetkräfte, sodass eine mechanische Kopplung nicht möglich ist. Danach erlauben die zumindest zwei Magnetelemente unterschiedlicher Polarität eine eineindeutige Zuordnung der Lage des Anschlussgehäuses an dem Gehäuse in gekoppeltem Zustand, wodurch die Kontaktelemente in richtiger Ausrichtung und Anordnung mit den Kontaktgegenelementen elektrisch verbunden werden.

Gemäß einer bevorzugten Weiterbildung der vorliegenden Erfindung wird ein Gegengehäuse vorgeschlagen, das mit dem Gehäuse verbunden ist und eine Anlagefläche aufweist, die zumindest angenähert bündig zu einer Außenfläche des Gehäuses vorgesehen ist. Das Gegengehäuse nimmt üblicherweise die Kontaktgegenelemente in sich auf und hält die zu den Kontaktgegenelementen gehäuseseitig vorgesehenen Anschlusskabel oder andere elektrische Leiter zur Weiterleitung des Leistungsstromes an den zumindest einen Verbraucher in dem Gehäuse. Dem Gegengehäuse kommt üblicherweise vor allem die Funktion zu, die Kontaktgegenelemente unverschieblich zu halten und deren Kontaktfläche an einer Außenseite verfügbar zu machen sowie mit dem eigentlichen Gehäuse verbunden zu werden. Das Anschlussgehäuse ist dementsprechend Komponente eines Kontaktpaares bestehend aus dem Anschlussgehäuse einerseits und dem Gegengehäuse andererseits. Dabei ist das Anschlussgehäuse üblicherweise mit dem Anschlusskabel verbunden, während von dem Gegengehäuse die Verkabelung innerhalb des Gehäuses zu dem Verbraucher, insbesondere dem Pumpaggregat abgeht. Es versteht sich, dass diese elektrischen Leiter nicht notwendigerweise unmittelbar mit dem Pumpaggregat elektrisch leitend verbunden sein müssen. Vielmehr erfolgt die Kontaktierung üblicherweise unter Zwischenschaltung einer Steuerung, die Steuerbefehle empfängt und die Weitergabe des Leistungsstromes an den Verbraucher von verschiedenen Einflussgrößen abhängig machen kann.

Das Gehäuse weist üblicherweise eine Öffnung auf, in welcher das Gegengehäuse eingesetzt ist. Das Gegengehäuse ist dabei insbesondere bei medizinischen Verwendungen bündig oder angenähert bündig zu der Außenfläche des Gehäuses, beispielsweise in einer durch das Gehäuse gebildeten Mulde, vorgesehen. So wird auch durch diese Ausgestaltung der gehäuseseitigen Komponenten der elektrischen Kontaktierung die Möglichkeit einer leicht zu reinigenden beziehungsweise zu sterilisierenden Ausgestaltung geschaffen.

Das Anschlussgehäuse und/oder das Gegengehäuse bestehen üblicherweise aus einem elektrisch isolierenden Material wie beispielsweise Keramik oder Kunststoff. So können die Kontaktelemente beziehungsweise Kontaktgegenelemente auf einfache Weise an dem entsprechenden Gehäuse befestigt und gehalten werden. Insbesondere bei einer mechanischen Kopplung mittels Magnetkraft ist es indes zu bevorzugen, die gehäuseseitige Anlagefläche durch eine elektrisch nichtleitende Beschichtung auszubilden, die außenseitig zumindest angenähert bündig oder bündig mit den Kontaktflächen der Kontaktgegenelemente vorgesehen ist. Diese Beschichtung ist üblicherweise eine Beschichtung aus einem dämpfenden Kunststoff, bevorzugt Silikon oder TPE. Die Beschichtung kann auf dem Gegengehäuse aufgebracht, beispielsweise aufgeklebt oder mittels Verbundspritzgießen daran vorgesehen sein. Die Außenfläche der Beschichtung und die Kontaktfläche der Kontaktgegenelemente, über welche diese elektrisch mit den Kontaktelementen kontaktiert werden, sind angenähert, bevorzugt vollständig flächenbündig, sodass sich gehäuseseitig eine ebene und glatte, teilweise durch die Beschichtung und teilweise durch die Kontaktflächen gebildete Oberfläche ergibt, die leicht zu reinigen ist. Es versteht sich, dass die Kontaktgegenelemente dazu üblicherweise abgedichtet durch die Beschichtung hindurchgeführt sind. Die Verbindung kann Ergebnis eines Verbundspritzgießens sein, bei welcher das Anschlussgehäuse aus einem Hartkunststoff zusammen mit den darin durch Umspritzen eingebetteten Kontaktgegenelementen, die zunächst die Oberfläche des Anschlussgehäuses überragen, mit einem die Beschichtung ausbildenden Kunststoff überspritzt wird. Auch kann die Beschichtung nachträglich aufgezogen werden, wobei Ausnehmungen zur Aufnahme der freien Endabschnitte der Kontaktelemente kleinere Abmessungen als die Kontaktelemente haben können, sodass diese in die Beschichtung eingepresst sind, wodurch die gewünschte Abdichtung erreicht wird. Auch kann die Beschichtung mit den Kontaktgegenelementen verklebt sein.

Im Hinblick auf einen Toleranzausgleich wird gemäß der vorliegenden Erfindung vorgeschlagen, die durch das Kontaktelement gebildete Kontaktfläche flächenmäßig kleiner als die zur Kontaktierung mit dem Kontaktelement vorgesehene Kontaktgegenfläche des Kontaktelementes vorzusehen. Während das Kontaktelement üblicherweise als Stift vorgesehen ist, ist die Kontaktgegenfläche um ein Vielfaches größer als die Stirnseite des Kontaktstiftes, sodass gewisse Toleranzen innerhalb der richtigen Positionierung des Anschlussgehäuses relativ zu dem Gehäuse möglich sind, ohne die gewünschte Übertragung des Leistungsstromes zu beeinträchtigen. Mit dieser Lösung wird insbesondere das Fehlen von Formschlusselementen kompensiert, die das Anschlussgehäuse gegenüber dem Gehäuse in vorbestimmter Weise ausrichten, indes im Hinblick auf die gewünschte glatte Oberfläche im Bereich der Kontaktierung vermieden werden sollten. Es versteht sich, dass die Größe der Kontaktgegenfläche so zu wählen ist, dass bei denkbaren Toleranzen immer bei einer mechanisch gehaltenen Kopplung von Anschlussgehäuse und Gehäuse das jeder Kontaktgegenfläche zugeordnete Kontaktelement mit der entsprechenden Kontaktgegenfläche elektrisch leitend kontaktiert ist. Die Toleranz und das Spiel lassen eine Bewegung zwischen der Kontaktfläche und der Kontaktgegenfläche zu, was eine Selbstreinigung der den elektrischen Kontakt bewirkenden Oberflächen begünstigt. Durch die Relativbewegung werden Verschmutzung oder metallische Oxide an den Oberflächen abgeschabt, sodass eine gute elektrische Kontaktierung dauerhaft gewährleistet ist.

Zur Überprüfung der richtigen Kontaktierung zur Übertragung des Leistungsstromes weist die erfindungsgemäße Lösung bevorzugt einen optischen Indikator auf, der den Status der Leistungsstromzufuhr anzeigt. Dieser optische Indikator ist üblicherweise eine Kontrollleuchte, die an dem Gehäuse und/oder dem Anschlussgehäuse vorgesehen sein kann. Im einfachsten Fall ist der optische Indikator als LED an dem Anschlussgehäuse vorgesehen und datenmäßig mit der Auswerteinheit gekoppelt, sodass bei Freischalten des Schalters zur Übertragung des Leistungsstromes zeitgleich auch die LED eingeschaltet wird.

Gemäß einer bevorzugten Weiterbildung der vorliegenden Erfindung sind die Kontaktelemente beziehungsweise die Kontaktgegenelemente zu unterschiedlicher Polarität und die Magnetelemente versetzt zu einer Mittellängsachse angeordnet, die üblicherweise durch das Anschlussgehäuse gegebenenfalls auch durch das Gegengehäuse gebildet und vorgegeben wird. Eine solche asymmetrische Ausgestaltung erhöht die elektrische Sicherheit, da aufgrund der Magnete eine eineindeutige Positionierung des Anschlussgehäuses an dem Gehäuse vorgegeben ist und die Kontaktelemente beziehungsweise Kontaktgegenelemente nicht auf der Mittellängsachse liegen, sodass bei der mechanischen Kopplung notwendigerweise auch das Kontaktelement mit dem richtigen Kontaktgegenelement kontaktiert wird.

Aus entsprechenden Überlegungen wird gemäß einer bevorzugten Weiterbildung der vorliegenden Erfindung vorgeschlagen, dass zu jeder Polarität zumindest zwei Kontaktelemente bzw. Kontaktgegenelemente vorgesehen sind, die bei einer Draufsicht auf die Kontaktfläche Eckpunkte einer Grundfläche definieren, wobei das Sensorelement bzw. das Sensorgegenelement innerhalb dieser Grundfläche bevorzugt asymmetrisch zu dieser Grundfläche und besonders bevorzugt an einem Rand der Grundfläche angeordnet ist. Auch diese Ausgestaltung verhindert zuvorderst eine falsche Verbindung der Kontaktelemente mit den Kontaktgegenelementen. Darüber hinaus erhöht die pro Polarität vorgesehene Anzahl an Kontaktelementen bzw. Kontaktgegenelementen die Sicherheit bei der Übertragung des Leistungsstroms. Die Grundfläche ist bevorzugt eine Rechteckfläche, wobei die Kontaktelemente bzw. Kontaktgegenelemente gleicher Polarität üblicherweise jeweils auf einer Gerade liegen, die eine Seitenfläche des Rechtecks ausbildet. Die Kontaktelemente bzw. Kontaktgegenelemente der anderen Polarität liegen gegenüberliegend dazu, wobei die parallelen Reihen von Kontaktelementen üblicherweise durch die längere der Seitenflächen des Rechtecks voneinander getrennt sind. Bei einer solchen Ausgestaltung liegen nicht alle Kontaktelement bzw. Kontaktgegenelemente auf einer Linie. Vielmehr sind, wie oben dargestellt, die Kontaktelemente bzw. die Kontaktgegenelemente einander gegenüberliegend und jeweils für sich auf gleichen Linien angeordnet. Auf einer dieser Ränder liegt üblicherweise das Sensorelement bzw. das Sensorgegenelement. Sämtliche Kontaktelemente liegen bevorzugt zwischen den Magnetelementen, wodurch die Gefahr einer fehlerhaften elektrischen Kontaktierung weiter reduziert wird. Auch wird mit dieser Anordnung verhindert, dass fremde Magnetstecker oder andere Ladestecker mit dem Gerät verbunden werden. Eine elektrische Überspannung mit Folgeschäden kann somit verhindert werden.

Gemäß einer bevorzugten Weiterbildung der vorliegenden Erfindung kann ein Kontaktelement bzw. ein Kontaktgegenelement in das Magnetelement integriert sein. Das Kontaktelement kann dabei von einem ringförmigen Magnetelement umgeben sein oder durch eine metallische Oberfläche des Magneten selbst ausgebildet sein. Die Ausgestaltung kann an dem Anschlussgehäuse und/oder gehäuseseits verwirklicht sein. Dabei kann durch das Kontaktelement selbst auch das Magnetelement gebildet sein, insbesondere, wenn es sich um ein elektromagnetisches Element handelt, welches durch Bestromung magnetisch wird.

Gemäß ihrem nebengeordneten Aspekt schlägt die vorliegende Erfindung ein Verfahren zum Anschließen eines Verbrauchers, beispielsweise einer motorgetriebenen medizinischen Saugpumpe mit einem Pumpaggregat zur Erzeugung eines Unterdrucks, an eine Stromquelle für den Leistungsstrom vor. Bei dem erfindungsgemäßen Verfahren wird zunächst zumindest eines der Kontaktelement zur Bestromung des Verbrauchers elektrisch mit gehäuseseitigen Kontaktgegenelement verbunden. Die Gehäuseseite ist dabei diejenige Seite der Kontaktierung, die dem Verbraucher, speziell der motorgetriebenen Saugpumpe zugeordnet ist. Die Verbindung erfolgt bei einem stromlos geschalteten Kontaktelement. Nachdem aufgrund eines von einem die Kontaktierung bestätigenden Sensors ein Signal abgegeben wurde, wird bei dem erfindungsgemäßen Verfahren der Leistungsstrom auf das Kontaktelement aufgeschaltet. Das Verfahren wird bevorzugt für sämtliche Kontaktelemente der Kontaktierung zwischen dem Verbraucher und dem Anschlussgehäuse zeitgleich durchgeführt, sodass der Leistungsstrom zur gleichen Zeit auf sämtliche bestromte Kontaktelemente aufgeschaltet wird. Der die Kontaktierung bestätigende Sensor kann ein unmittelbar die elektrische Kontaktierung zwischen dem Kontaktelement und dem Kontaktgegenelement erfassender Sensor sein. Mit anderen Worten detektiert der Sensor das elektrische Anliegen von Kontaktelement und Kontaktgegenelement. Alternativ kann der Sensor mittelbar auf die Kontaktierung durch Detektion der richtigen Positionierung des Anschlussgehäuses relativ zu dem Gehäuse des Verbrauchers schließen.

Weitere Einzelheiten und Vorteile der vorliegenden Erfindung ergeben sich aus der nachfolgenden Beschreibung von Ausführungsbeispielen in Verbindung mit der Zeichnung. In dieser zeigen:
- Fig. 1: eine perspektivische Seitenansicht einer medizinischen Saugpumpe zum Absaugen von Exsudat vor der elektrischen Kontaktierung;
- Fig. 2: eine perspektivische Seitenansicht einer medizinischen Saugpumpe mit Brustpumpen für das Absaugen von Muttermilch nach der Kontaktierung;
- Fig. 3: eine Draufsicht auf die Anlagefläche eines Ausführungsbeispiels eines Anschlussgehäuses;
- Fig. 4: eine Seitenansicht des Anschlussgehäuses nach Fig. 3;
- Fig. 5: eine rückseitige Draufsicht des Anschlussgehäuses nach den Fig. 3 und 4;
- Fig. 6: perspektivische, teilweise geschnittene Seitenansicht des Anschlussgehäuses nach den Fig. 3 bis 5 beim Koppeln mit dem Gehäuse der Saugpumpe; und.
- Fig. 7: ein vergrößertes Detail VII gemäß Fig. 6.

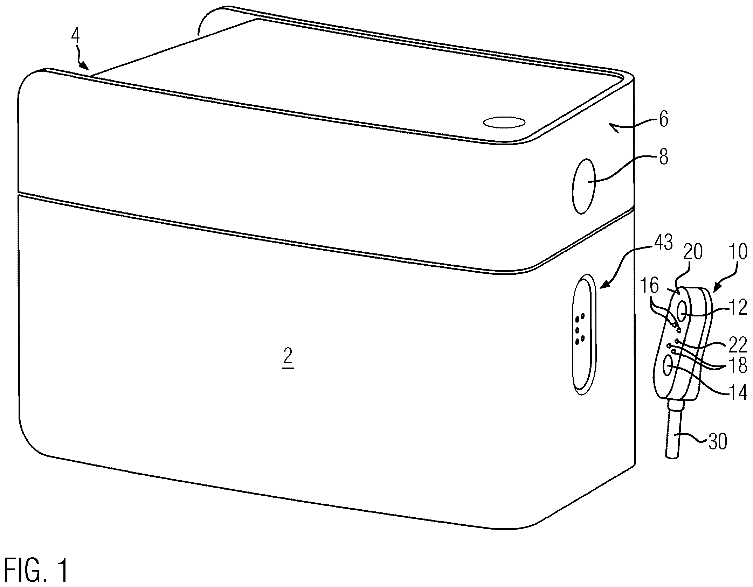

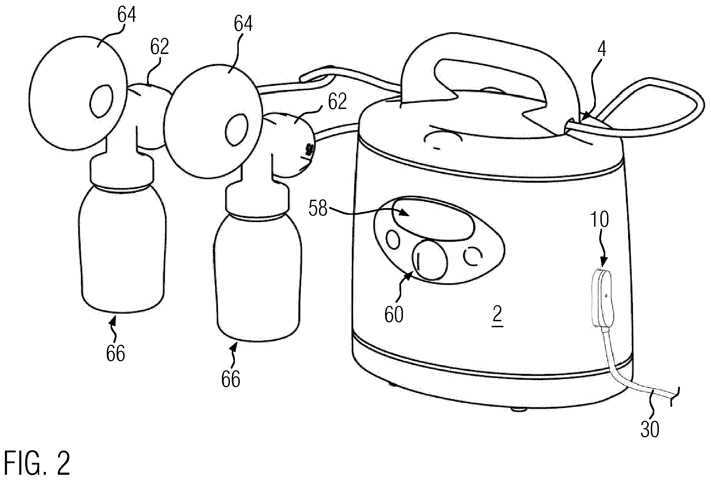

Die Fig. 1 und 2 zeigen jeweils Ausführungsbeispiele von medizinischen Saugpumpen anhand einer Exsudatpumpe (Fig. 1) bzw. einer Saugpumpe für das Abpumpen von Muttermilch aus der weiblichen Brust (Fig. 2). Die in beiderseitigen Beispielen weisen in ihrem Gehäuse 2 jeweils ein zeichnerisch nicht dargestelltes Pumpaggregat auf, mit welchem ein Unterdruck erzeugt wird, der beispielsweise an einer Schnittstelle 4 anliegt, an der beispielsweise ein Unterdruckschlauch angeschlossen werden kann. Diese Schnittstelle 4 ist in Fig. 2 zeichnerisch verdeutlicht. Bei dem in Fig. 1 gezeigten Ausführungsbeispiel befindet sich die Schnittstelle 4 an einer Rückseite, deren Details nicht zu erkennen sind. Die Schnittstelle 4 kommuniziert hier mit einem mechanisch mit dem Gehäuse 2 koppelbaren Exsudatbehälter, der als Einwegteil zum Auffangen von Exsudat angepasst ausgebildet ist. Die Rückseite ist dabei parallel und gegenüberliegend zu einer mit Bezugszeichen 6 berechneten Vorderseite vorgesehen, die zur Kontaktierung mit dem Leistungsstrom angepasst ausgebildet und mit einem Netzschalter 8 versehen ist.

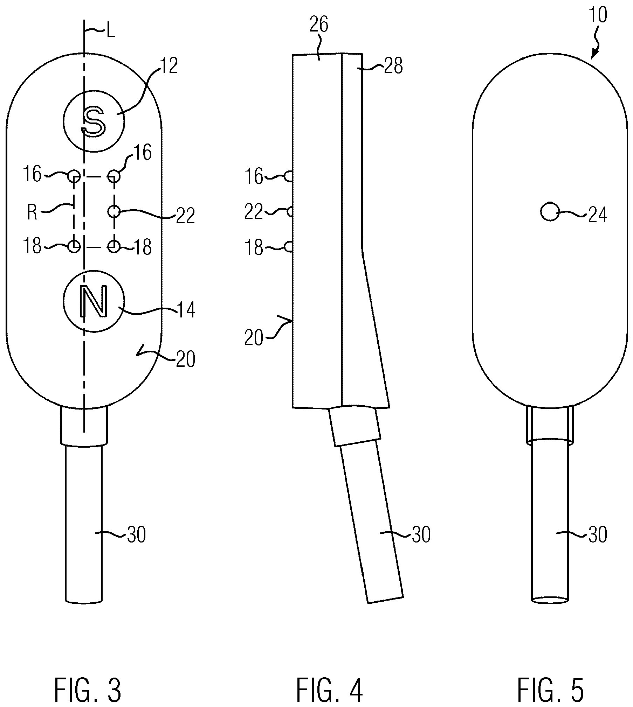

Die Kontaktierung mit dem Leistungsstrom erfolgt bei beiden Ausführungsbeispielen über ein Anschlussgehäuse 10, das mit zwei Magnetelementen 12, 14 versehen ist, die jeweils unterschiedlichen Polen S, N zugeordnet sind, und mehrere Kontaktelemente 16, 18 zwischen sich aufnehmen, die eine Anlagefläche 20 überragen. Die Kontaktelemente 16 sind einer Polarität des Leistungsstromes zugeordnet, die Kontaktelemente 18 der anderen Polarität. Wie Fig. 3 verdeutlicht, spannen die Kontaktelemente 16, 18 eine Rechteckfläche Rauf. Auf dem in Fig. 3 rechten Rand dieses Rechtecks liegt ein Sensorelement 22, welches vorliegend als Sensorstift ausgebildet ist. So liegen die Kontaktelemente 16, 18 und der Sensorstift 22 in der Anlagefläche 22 frei. Tatsächlich überragen die Kontaktelemente 16, 18 und der Sensorstift 22 die Anlagefläche 20. Wie Fig. 4 vermittelt, stehen die Kontaktelemente 16, 18 mit ihren freien Kontaktflächen 23 weiter von der Anlagefläche 20 ab als der Sensorstift 22.

Die Rückseite des Anschlussgehäuses 10 ist in Fig. 5 verdeutlicht. Dort liegt als optischer Indikator eine LED 24 frei. Das Anschlussgehäuse 10 ist vorliegend zweiteilig ausgebildet, wobei zwischen einem Unterteil 26 und einem Oberteil 28 ein Anschlusskabel 30 in das Anschlussgehäuse 10 geführt ist. Das Anschlusskabel 30 hat an seinem anderen Ende einen Stecker für die Steckkontaktierung in einer konventionellen Steckdose für den Anschluss 230/110 V und kann dort mit einem elektrischen Vorschaltgerät versehen sein, welcher die Spannung auf 12 V transformiert und mit dieser Spannung den Leistungsstrom in das Anschlussgehäuse 10 einspeist.

Wie insbesondere die Zusammenschau der Fig. 3 und 4 vermittelt, ist die Anlagefläche 20 des Anschlussgehäuses 10 vollständig eben. Die Magnetelemente 12, 14 sind absatzfrei in die Anlagefläche 20 eingelassen. Auch ansonsten ist das Anschlussgehäuse 10 ohne Hinterschnitte und glatt ausgebildet. Es hat eine rechtecksförmige Grundfläche mit abgerundeten Ecken. Die Gestaltung des Anschlussgehäuses 10 vermeidet Kanten oder Hinterschnitte, in denen sich Verschmutzung und Bakterien einnisten können. Auch ein das Anschlusskabel 30 führender und als Teil des Anschlussgehäuses 10 vorgesehener Stutzen kann konisch bzw. mit einem Radius in das eigentliche Anschlussgehäuse 10 übergehen, um der zuvor erwähnten Anforderung bestmöglich zu genügen.

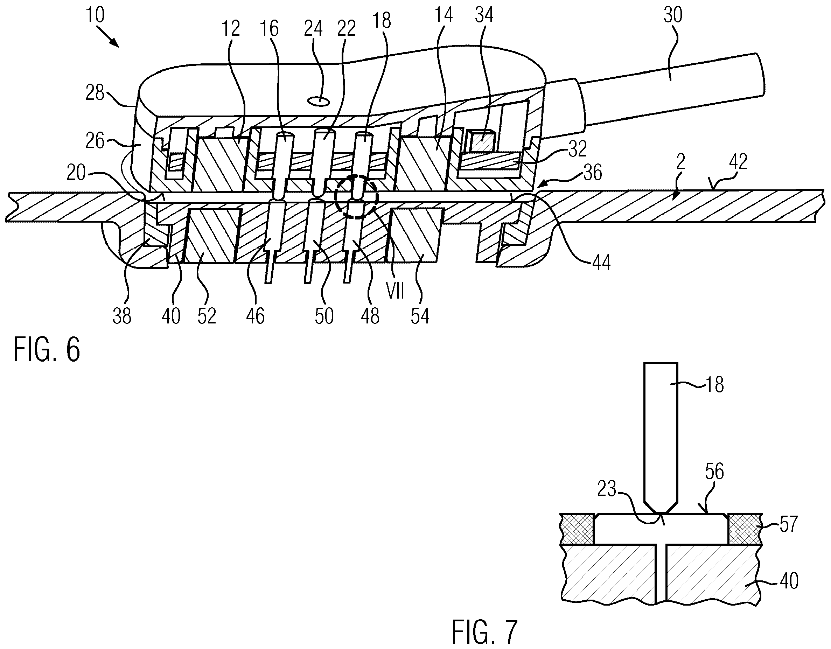

Die teilweise geschnittene Darstellung des Anschlussgehäuses 10 nach Figur 6 verdeutlicht schematisch einige in dem Anschlussgehäuse 10 vorgesehenen Komponenten. Eine Platine 32 ist elektrisch leitend mit den Kontaktelementen 16, 18 und dem Sensorstift 22 verbunden. Die Platine 32 trägt einen Schalter 34, der mit einer durch die bestückte Platine 32 selbst gebildeten Auswerteinheit datenmäßig verbunden ist.

Die Fig. 6 verdeutlicht die Annäherung des Anschlussgehäuses 10 an das Gehäuse 2 im Rahmen der Kontaktierung. Das Gehäuse 2 weist eine Aussparung 36 auf, in welches unter Zwischenlage einer elastomeren Dichtung 38 ein Gegengehäuse 40 eingesetzt ist. Die Oberfläche der Dichtung 38 und die Oberfläche des Gegengehäuses 40 liegen leicht innerhalb zu einer Außenfläche 42 des Gehäuses und bilden in einer um weniger als 3 mm nach innen versetzten Mulde 43 eine mit Bezugszeichen 44 gekennzeichnete gehäuseseitige Anlagefläche aus, deren Abmessungen und Form in etwa der Abmessung und Form der Anlagefläche 20 des Anschlussgehäuses 10 entspricht. Es versteht sich, dass die Mulde 43 geringfügig größer als die Anlagefläche 20 ist.

In der Anlagefläche 44 liegen zur Kontaktierung mit den Kontaktelementen 16, 20 vorgesehene Kontaktgegenelemente 46, 48 frei. Als Sensorgegenelement ist ein mit Bezugszeichen 50 gekennzeichneter Sensorgegenstift vorgesehen. Korrespondierend zu den Magnetelementen 12, 14 sind die Magnetelemente 12 bzw. 14 jeweils anziehende Magnetgegenelemente 52, 54 innerhalb des Gegengehäuses 40 aufgenommen und von diesem gehalten.

Durch die Kraft der beiderseitigen Magnete 12, 52 bzw. 14, 54 wird das Anschlussgehäuse 10 bei Annäherung an das Gehäuse 2 in Richtung auf das Gegengehäuse 40 gezogen. Durch die Magnetkraft erfolgt eine exakte Positionierung des Anschlussgehäuses 10 relativ zu dem Gehäuse 2, sodass die Kontaktelemente 16, 18 und der Sensorstift 20 auf Kontaktgegenfläche 56 fluchten, die innerhalb der Anlagefläche 44 durch die Kontaktgegenelemente 46, 48 bzw. den Sensorgegenstift 50 gebildet sind.

Ein Beispiel einer Kontaktgegenfläche 56 ist in Fig. 7 verdeutlicht. Ersichtlich läuft der das Kontaktelement ausbildende Kontaktstift 18 an seinem freien Ende spitz zu und bildet eine gegenüber dem Querschnitt des Kontaktstiftes 18 verminderte, gleichwohl im Wesentlichen kreisrunde Kontaktfläche 23 aus. Die durch das Kontaktgegenelement 48 gebildete Kontaktgegenfläche 56 ist wesentlich größer, sodass auch bei einem relativen Versatz des Anschlussgehäuses 10 relativ zu der idealen Position eine elektrische Kontaktierung zwischen den Kontaktelementen 16, 18 und den zugehörigen Kontaktgegenelementen 46, 48 sicher gewährleistet ist.

Wie die Fig. 4 und 6 verdeutlichen liegt das freie Ende des Sensorstiftes 22 näher an der Anlagefläche 20 als die zugeordneten Kontaktflächen 23 der Kontaktelemente 16, 18. Diese liegen in einer Ebene, die parallel zu der Ebene der Anlagefläche 20 verläuft. Zwischen dieser Ebene und der Anlagefläche 20 liegt das freie Ende des Sensorstiftes 22. So ragt der Sensorstift 22 in Ankoppelrichtung bei Annäherung des Anschlussgehäuses 10 an das Gehäuse 2 weniger weit von der Anlagefläche 20 ab als die Kontaktelemente 16, 18. Der Sensorstift 22 ist dementsprechend ein nachlaufender Kontaktstift, der erst auf die Kontaktgegenfläche des zugeordneten Sensorgegenstiftes 50 trifft, nachdem zuvor üblicherweise sämtliche Kontaktelemente 16, 18 mit den zugeordneten Kontaktgegenelementen 46, 48 kontaktiert worden sind. Das Kontaktgegenelement 48 kann beispielsweise mit dem Sensorgegenelement 50 gehäuseseitig kurzgeschlossen werden. Beispielsweise durch Messen des elektrischen Widerstandes von dem Kontaktelement 18 in das Kontaktgegenelement 48 und von dort in das Sensorgegenelement 50 zu dem Sensorelement 22 kann die Kopplung des Anschlussgehäuses 10 an das Gehäuse 2 detektiert werden. Die auf der Platine 32 vorgesehene Schaltung gibt dann ein Signal an den Schalter 34, der den Leistungsstrom freischaltet, um über die Kontaktelemente 16, 18 den Leistungsstrom in das Gehäuse 2 einzuspeisen. Zeitgleich wird die LED 24 angeschaltet.

Die Schnittansicht gemäß Fig. 6 verdeutlicht auch die im Wesentlichen absatzfreie gehäuseseitige Gestaltung. Das Gehäuse 2 geht über eine durch die Dichtung 38 gebildete Dichtlippe nahezu gleichmäßig und absatzfrei in die Oberfläche des Gegengehäuses 40 über. Auch insofern wird es verhindert, dass sich an dieser Stelle der elektrischen Kontaktierung Bakterien oder Verschmutzung einnisten. Zusätzlich kann die zu dem Anschlussgehäuse 10 weisende Außenfläche des Gegengehäuses mit einer weichelastischen Beschichtung versehen sein, sodass das durch die Magnetkraft angezogene Anschlussgehäuse 10 nicht ungedämpft gegen das Gehäuse 2 schlägt.

Die Fig. 7 verdeutlicht in diesem Zusammenhang eine fest auf dem Gegengehäuse 40 aufliegende, elektrisch nicht-leitende Beschichtung 57, deren Oberfläche höhengleich und bündig zu der Kontaktgegenfläche 56 vorgesehen ist. Diese Beschichtung 57 kann einteilig mit der Dichtung 38 ausgebildet sein. Die Beschichtung 57 ist bevorzugt zumindest annähernd höhengleich zu der die Anlagefläche 40 umgebenden Außenfläche 42 vorgesehen.

Die erfindungsgemäße Lösung mit einem Schalter, der erst den Leistungsstrom auf die Kontaktelemente schaltet, wenn diese an den Kontaktgegenelementen anliegen und mit diesen kontaktiert sind, und dementsprechend den Leistungsstrom abschaltet, bevor die Kontaktelemente von den Kontaktgegenelementen gentrennt sind, verhindert Funkenbildung bzw. einen Lichtbogen vor allem beim Trennen der Kontaktierung der Kontaktelemente, wodurch die Lebensdauer der elektrischen Kontaktierung erhöht wird. Die Kontaktflächen brennen nicht aufgrund eines Lichtbogens zwischen den noch nicht kontaktierten Kontaktelementen ab. Auch bleiben die durch die Kontaktelemente 16, 18 gebildeten elektrischen Kontakte spannungsfrei, sodass eine gesundheitliche Beeinträchtigung und/oder Gefahr durch eine an den Kontaktelementen 16, 18 anliegende Spannung nicht gegeben ist. Die Spannung wird erst aufgeschaltet, wenn die Kontaktelemente 16, 18 mit den zugeordneten Gegenelementen 46, 48 kontaktiert sind. Diese liegen entweder eben in der Oberfläche des Gehäuses 2 oder in einer leichten Vertiefung. Jedenfalls aber überragt bei dem gezeigten Ausführungsbeispiel das Anschlussgehäuse 10 die Kontaktelemente 16, 18 umfänglich, sodass nach einer mechanischen Kopplung des Anschlussgehäuses 10 an dem Gehäuse 2 keine Berührung der Kontaktelemente 16, 18 durch einen Benutzer und/oder eine Kontaktierung mit elektrisch leitenden Medien, wie Flüssigkeit oder Gas, möglich ist. Insofern kann auch die weichelastische Beschichtung auf der Außenfläche des Gegengehäuses 40 eine zusätzliche Abdichtung und damit verbesserte elektrische Sicherheit bewirken.

Bei erfolgter Kopplung, in der Regel nach Freischalten des Leistungsstromes, wird auch die LED 24 angeschaltet, sodass dem Benutzer die erfolgte Kontaktierung angezeigt wird.

Durch die Magnetelemente 12, 14 und die zugeordneten Magnetgegenelemente 52, 54 ist eine eineindeutige Positionierung gegeben. Darüber hinaus spannen die Kontaktelemente 16, 18 ein Rechteck auf, welches in Fig. 3 mit Bezugszeichen R gekennzeichnet ist. Über die längere Seite des Rechtecks R sind die Kontaktelemente 16 der einen Polarität von den Kontaktelementen 18 der anderen Polarität relativ weit entfernt. Dazwischen liegt das Sensorelement 22. Das Rechteck R befindet sich zwischen den Magnetelementen 12, 14. Wie ersichtlich liegt die Mittellängsachse des Rechtecks R exzentrisch zu einer Mittellängsachse L des Anschlussgehäuses 10. Aufgrund der länglichen Ausgestaltung des Anschlussgehäuses 10 und der leicht nach hinten zurück versetzten Anlagefläche 44 zur gehäuseseitigen Aufnahme desselben ergibt sich mit den Magnetkräften der Magnetelemente 12, 14 eine eineindeutige Positionierung, die aufgrund der polverschiedenen Magnetkräfte nicht vertauscht werden kann.

Durch den Umstand, dass die Kontaktgegenflächen 56 jeweils größer als die durch die Kontaktelemente 16, 18 und dem Sensorstift 22 gebildeten Kontaktflächen 23 sind, wird eine sichere Kontaktierung begünstigt. Ersichtlich liegen die Kontaktelemente 16 der einen Polarität und die Kontaktelemente 18 der anderen Polarität auf einer Linie, die durch die gegenüberliegenden schmaleren Seitenflächen des Rechtecks R gebildet ist. Die Kontaktelemente unterschiedlicher Polarität liegen dementsprechend nicht auf der gleichen Linie. Die auf der Anlagefläche 20 gegebene Kriechstrecke von den Kontaktelementen 16 einer Polarität zu den Kontaktelementen 18 der anderen Polarität ist vergrößert. Der dazwischen liegende Raum wird für die Anordnung des Sensorelementes 22 genutzt, der auf einem Rand des Rechtecks R und damit vorliegend exakt auf einer Verbindungslinie zwischen dem einen der Kontaktelemente 16 und dem anderen der Kontaktelemente 18 liegt.

Das in Fig. 2 verdeutlichte Ausführungsbeispiel einer motorgetriebenen Saugpumpe umfasst das Gehäuse 2, welches die zuvor beschriebene Anschlussmöglichkeit für die Kopplung des Anschlussgehäuses 10 ausbildet und ferner mit einem Display 58 und Steuerelementen 60 für die Eingabe von Betriebsparametem versehen ist. Vorliegend sind zwei Verbindungsteile 62 mit jeweils einer Brusthaube 64 zu jeweils einer Flasche 66 über die Schnittstelle 4 mit der Saugseite der motorgetriebenen medizinischen Saugpumpe verbunden.

### Bezugszeichenliste

- 2: Gehäuse
- 4: Schnittstelle
- 6: Vorderseite
- 8: Netzschalter
- 10: Anschlussgehäuse
- 12: Magnetelement, Süd
- 14: Magnetelement, Nord
- 16: Kontaktelement
- 18: Kontaktelement
- 20: Anlagefläche
- 22: Sensorelement/Sensorstift
- 24: LED
- 26: Unterteil
- 28: Oberteil
- 30: Anschlusskabel
- 32: Platine
- 34: Schalter
- 36: Aussparung
- 38: Dichtung
- 40: Gegengehäuse
- 42: Außenfläche
- 43: Mulde
- 44: Anlagefläche
- 46: Kontaktgegenelement
- 48: Kontaktgegenelement
- 50: Sensorgegenstift
- 52: Magnetgegenelement
- 54: Magnetgegenelement
- 56: Kontaktgegenfläche
- 57: Beschichtung
- 58: Display
- 60: Steuerelement
- 62: Verbindungsteil
- 64: Brusthaube
- 66: Flasche
- R: Rechteck
- L: Mittellängsachse des Anschlussgehäuses 10

## Patentansprüche

1. Motorgetriebene medizinische Saugpumpe mit einem Pumpaggregat zur Erzeugung eines Unterdrucks, das in einem Gehäuse (2) aufgenommen ist, und mit einem mit dem Gehäuse mechanisch koppelbaren Anschlussgehäuse (10), das über ein Anschlusskabel (30) an eine Stromquelle anschließbar ist und Kontaktelemente (16; 18) zur Bestromung des Pumpaggregats mit einem Leistungsstrom aufweist, die bei an dem Gehäuse (2) gekoppeltem Anschlussgehäuse (10) elektrisch leitend mit gehäuseseitig vorgesehenen, zu dem Pumpaggregat führenden Kontaktgegenelementen (46; 48) verbunden sind,
**dadurch gekennzeichnet,**
**dass** ein Sensor (22), eine Auswerteinheit (32) und ein Schalter (34) vorgesehen sind, dass der Sensor (22) zur Überprüfung der Kopplung des Anschlussgehäuses (10) eingerichtet ist, dass der Schalter (34) eine Leistungsstromzufuhr zu zumindest einem der Kontaktelemente (16; 18) schaltet und dass die Auswerteinheit (32) so datenmäßig mit dem Sensor (22) und dem Schalter (34) verbunden ist, dass der Schalter (34) bei einem die Kopplung bestätigenden Signal des Sensors (22) das Kontaktelement (16; 18) mit der Leistungsstromzufuhr verbindet und beim Ausbleiben eines die Kopplung bestätigenden Signals des Sensors (22) die Leistungsstromzufuhr trennt.

2. Motorgetriebene medizinische Saugpumpe nach Anspruch 1, **dadurch gekennzeichnet, dass** der Sensor ein Sensorelement (22) aufweist, dem gehäuseseitig ein Sensorgegenelement (50) zugeordnet ist, dass das Sensorelement (22) abhängig von der relativen Lage zu dem Sensorgegenelement (50) ein die Kopplung bestätigendes Signal abgibt, wobei zumindest eines der Kontaktelemente (16; 18) und das Sensorelement (22) so in dem Anschlussgehäuse (10) angeordnet sind, dass bei mechanischer Koppelung des Anschlussgehäuses (10) an dem Gehäuse (2) zunächst das Kontaktelement (16) mit dem zugeordneten Kontaktgegenelemente (46) elektrisch verbunden wird und erst danach das Sensorelement (22) relativ zu dem Sensorgegenelement (50) so positioniert wird, dass ein die Kopplung bestätigendes Signal abgesetzt wird.

3. Motorgetriebene medizinische Saugpumpe nach Anspruch 1 oder 2, **dadurch gekennzeichnet, dass** das Anschlussgehäuse (10) mittels Magnetkraft an dem Gehäuse gehalten ist, wobei vorzugsweise das Anschlussgehäuse (10) zumindest zwei Magnetelemente (12, 14) aufweist, die mit unterschiedlichen Polen an einer im gekoppelten Zustand dem Gehäuse (2) gegenüberliegenden Anlagefläche (20) des Anschlussgehäuses (10) zur eineindeutigen Positionierung des Anschlussgehäuses (10) relativ zu dem Gehäuse (2) wirksam sind.

4. Motorgetriebene medizinische Saugpumpe nach einem der vorherigen Ansprüche, **dadurch gekennzeichnet, dass** das Anschlussgehäuse (10) und/oder das Gehäuse (2) eine absatzfreie Anlagefläche (20; 44) aufweisen, an der das jeweils andere von dem Anschlussgehäuse (10) und dem Gehäuse (2) im gekoppelten Zustand anliegt.

5. Motorgetriebene medizinische Saugpumpe nach einem der vorherigen Ansprüche, **gekennzeichnet durch** ein Gegengehäuse (40), das mit dem Gehäuse (2) verbunden ist und eine im gekoppelten Zustand an dem Anschlussgehäuse (10) anliegende Anlagefläche (44) aufweist, die zumindest angenähert bündig zu einer Außenfläche (42) des Gehäuses (2) vorgesehen ist.

6. Motorgetriebene medizinische Saugpumpe nach einem der vorherigen Ansprüche, **dadurch gekennzeichnet, dass** die gehäuseseitige Anlagefläche durch eine elektrisch nicht-leitenden Beschichtung ausgebildet ist, die außenseitig zumindest angenähert bündig mit Kontaktgegenflächen (56) der Kontaktgegenelemente (46; 48) vorgesehen ist.

7. Motorgetriebene medizinische Saugpumpe nach einem der vorherigen Ansprüche, **dadurch gekennzeichnet, dass** eine durch das Kontaktelement (16; 18) gebildete Kontaktfläche (23) flächenmäßig kleiner als eine zur Kontaktierung mit dem Kontaktelement vorgesehene Kontaktgegenfläche (56) des Kontaktgegenelementes (46; 48) ist.

8. Motorgetriebene medizinische Saugpumpe nach einem der vorherigen Ansprüche **gekennzeichnet durch** einen optischen Indikator (24) zur Anzeige eines Status der Leistungsstromzufuhr.

9. Motorgetriebene medizinische Saugpumpe nach einem der vorherigen Ansprüche **dadurch gekennzeichnet, dass** die Kontaktelemente (16; 18) bzw. Kontaktgegenelemente (46; 48) zu unterschiedlicher Polarität und die Magnetelemente (12, 14; 52, 54) versetzt zu einer Mittellängsachse (L) des Anschlussgehäuses (10) bzw. des Gegengehäuses (40) angeordnet sind.

10. Motorgetriebene medizinische Saugpumpe nach einem der vorherigen Ansprüche **dadurch gekennzeichnet, dass** die Kontaktelemente (16; 18) federnd in dem Anschlussgehäuse (10) abgestützt sind.

11. Motorgetriebene medizinische Saugpumpe nach einem der vorherigen Ansprüche soweit von Anspruch 2 abhängig, **dadurch gekennzeichnet, dass** zu jeder Polarität zumindest zwei Kontaktelemente (16; 18) bzw. Kontaktgegenelemente (46; 48) vorgesehen sind, die bei einer Draufsicht auf die Kontaktfläche Eckpunkte einer Grundfläche (R) definieren, und dass das Sensorelement (22) bzw. das Sensorgegenelement (50) innerhalb dieser Grundfläche (R), bevorzugt asymmetrisch zu dieser Grundfläche (R), besonders bevorzugt auf einem Rand der Grundfläche (R) angeordnet ist.

12. Motorgetriebene medizinische Saugpumpe nach einem der vorherigen Ansprüche, **dadurch gekennzeichnet, dass** sämtliche Kontaktelemente (16; 18) bzw. Kontaktgegenelemente (46; 48) zwischen den Magnetelementen (12, 14; 52, 54) vorgesehen sind.

13. Motorgetriebene medizinische Saugpumpe nach einem der vorherigen Ansprüche, **dadurch gekennzeichnet, dass** nicht alle Kontaktelemente (16; 18) bzw. Kontaktgegenelemente (46; 48) in einer Linie angeordnet sind.

14. Motorgetriebene medizinische Saugpumpe nach einem der vorherigen Ansprüche, **dadurch gekennzeichnet, dass** die Kontaktelemente (16; 18) bzw. Kontaktgegenelemente (46; 48) im nicht-gekoppelten Zustand die ihnen zugeordneten Anlagefläche (20; 44) durchragen.

15. Motorgetriebene medizinische Saugpumpe nach einem der vorherigen Ansprüche, **dadurch gekennzeichnet, dass** zumindest ein Kontaktelement (16; 18) bzw. Kontaktgegenelement (46; 48) in das Magnetelement integriert ist.

16. Verfahren zum Anschließen einer motorgetriebenen medizinischen Saugpumpe mit einem Pumpaggregat zur Erzeugung eines Unterdrucks, das in einem Gehäuse (2) aufgenommen ist, an eine Stromquelle für einen Leistungsstrom, bei dem in einem Anschlussgehäuse (10) gehaltene und zu der Stromquelle führende Kontaktelemente (16; 18) zur Bestromung des Pumpaggregats elektrisch mit gehäuseseitigen Kontaktgegenelementen (46; 48) bei zumindest einem stromlos geschalteten Kontaktelement (16; 18) verbunden werden und danach aufgrund eines von einem die entsprechende Kontaktierung bestätigenden Sensor (22) abgegebenen Signals der Leistungsstrom auf das Kontaktelement (16; 18) aufgeschaltet wird.
